# EUROPEAN PATENT APPLICATION

(11) **EP 1 568 702 A1**
(43) Date of publication of application: **31.08.2005**
(21) Application number: 02799140.5
(22) Date of filing: 12.11.2002
(51) Int. Cl.: C07F 15/00, C07D 211/76, C07D 241/12, C07D 241/18, C07D 265/30, C07D 295/02, A61K 31/496, A61K 31/535, A61P 35/00

(54) **PALLADIUM COMPLEXES WITH HETEROCYCLIC LIGANDS**

(71) Applicant: ASGL - Farmatsevticjeskie Innovatsii, Zaptryote Aktsionernoe Obschestvo, St. Petersburg, 197022 (RU); Zakrytoe Aktsionernoe Obschestvo "ASGL-Issledovatelskie Laboratorii", St. Petersburg 197022 (RU)
(72) Inventor: EFIMENKO, Inessa Alexandrovna, Moscow, 119454 (RU); IVANOVA, Nina Alexandrovna, Khimki, 141406 (RU); LOKSHIN, Boris Veniaminovich, Moscow, 117133 (RU)
(74) Representative: Partio, Erja
(86) International application number: PCT/RU2002/000495
(87) International publication number: WO 2004/043975

(57) **Abstract**

The invention relates to novel chemical compounds, in particular palladium complexes with heterocyclic ligands of general formula I, 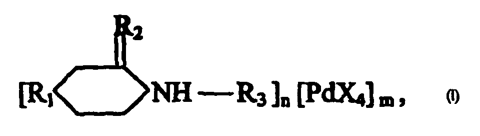 wherein R₁ is NH₂, O or CH₂; R₂ is 2H or O; R₃ is H, CH₃, CH₂-CH₂-NH₃ or (CO)-CH₃; X is Cl or Br; when R₁ is NH₂ or O; R₂ is 2H; R₃ is H, CH₃, CH₂-CH₂-NH₃ or (CO)-CH₃, then n = 1, and m = 1; when R₁ is O or CH₂; R₂ is O or 2H; R₃ is H, CH₃ or (CO)-CH₃, then n = 2, and m = 1; when R₁ is NH₂; R₂ is 2H; R₃ is CH₂-CH₂-NH₃, then n = 2, and m = 3; which compounds have pharmacological activity, in particular anti-tumor activity. The compounds of the invention are a higher activity and **characterized by** a lower toxicity as compared with anti-tumor platinum complex preparations.

## Description

### FIELD OF THE INVENTION

The invention relates to novel chemical compounds, in particular palladium complexes with heterocyclic ligands of general formula I, 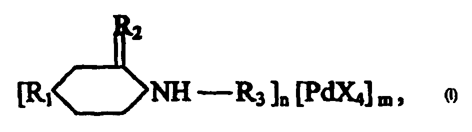 wherein
R₁ is NH₂, O or CH₂;
R₂ is 2H or O;
R₃ is H, CH₃, CH₂-CH₂-NH₃ or (CO)-CH₃;
X is Cl or Br;
when R₁ is NH₂ or O, R₂ is 2H, R₃ is H, CH₃, CH₂-CH₂-NH₃ or (CO)-CH₃, then n = 1, and m = 1;
when R₁ is O or CH₂, R₂ is O or 2H, R₃ is H, CH₃ or (CO)-CH₃, then n = 2, and m = 1;
when R₁ is NH₂, R₂ is 2H, R₃ is CH₂-CH₂-NH₃, then n = 2, and m = 3;
which compounds have pharmacological activity.

### DECRIPTION OF THE PRIOR ART

Platinum (II) complex compounds, such as platin, cysplatin and carboplatin, having anti-tumor activity are known in the prior art (see Mashkovsky M.D. Lekarstvennye Sredstva. 13^{th} edition, Kharkov, "Torsing", 1997, v. II, pages 458-460).

The compounds are close by the mechanism of action to alkylating compounds; they interact with DNA to form inter- and intramolecular cross-links. The mechanism of anti-tumor action of the platinum preparations is based on their ability to bifunctionally alkylate DNA strands, which leads to long-lasting inhibition of the biosynthesis of nucleic acids and to cell death.

The known platinum preparations are highly toxic. A serious side effect of the platinum preparations is nephrotoxicity. Among other adverse reactions induced by platinum preparations, leucopenia, nausea and emesis shall be noted.

Furthermore, since the known preparations are highly toxic and easily absorbed into the bloodstream by transdermal and mucosal routs, they are harmful to the health of those who are engaged in production of these preparations. It is necessary to handle them using rubber gloves and respirators.

Yet another important disadvantage of the known preparations is caused by the high cost of platinum, which makes such platinum preparations very expensive.

Thus there is a great need for new platinum metal preparations having a high degree of anti-tumor activity and characterized by lower toxicity.

### SUMMARY OF THE INVENTION

The principle object of the present invention is to provide novel chemical compounds which are biologically active, in particular having anti-tumor activity and characterized by low toxicity.

With this principle object in view, there are proposed novel chemical compounds which represent palladium complexes with heterocyclic ligands of general formula I: 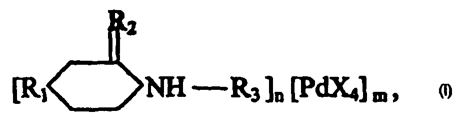 wherein
R₁ is NH₂, O or CH₂;
R₂ is 2H or O;
R₃ is H, CH₃, CH₂-CH₂-NH₃ or (CO)-CH₃;
X is Cl or Br;
when R₁ is NH₂ or O, R₂ is 2H, R₃ is H, CH₃, CH₂-CH₂-NH₃ or (CO)-CH₃, then n = 1, and m = 1;
when R₁ is O or CH₂, R₂ is O or 2H, R₃ is H, CH₃ or (CO)-CH₃, then n = 2, and m = 1;
when R₁ is NH₂, R₂ is 2H, R₃ is CH₂-CH₂-NH₃, then n = 2, and m = 3.

According to the invention, the novel compounds have a high anti-tumor activity and low toxicity.

In one preferred embodiment of the invention, compounds of general formula I represent palladium complexes of the cation-anion type comprising the [PdCl₄]²⁻ anion.

A process for preparation of the compounds comprises two steps.

In the first step, an organic ligand in solution is protonated with a hydrohalic acid. In the second step, the protonated ligand in solution is reacted with a palladium compound at a stoichiometric ratio of the reagents at a temperature from 30°C to 80°C followed by isolating the desired product from the solution.

The compounds of the invention can be prepared in both aqueous and non-aqueous medium.

In particular, the following specific compounds indicated in Table 1 have been prepared.

**Table 1**

| Compound | Formula | Ligand |
|---|---|---|
| I | [C₄H₁₂N₂][PdCl₄] | piperazine |
| II | [C₅H₁₄N₂][PdCl₄] | N-methyl-piperazine |
| III | [C₆H₁₄N₂O][Pd Cl₄] | 4-acetyl-piperazine |
| IV | [C₆H₁₈N₃]₂[PdCl₄]₃ | 4-aminoethyl-piperazine |
| V | [C₄H₁₀NO]₂[PdCl₄] | morpholine |
| VI | [C₄H₁₀NO]₂[PdBr₄] | morpholine |
| VII | [C₅H₁₂NO]₂[PdCl₄] | 4-methyl-morpholine |
| VIII | [C₆H₁₂NO₂]₂[PdCl₄] | 4-acetyl-morpholine |
| IX | [C₆H₁₆N₂O][PdCl₄]₃ · H₂O | 4-aminoethyl-morpholine |
| X | [C₅H₁₀NO]₂[PdCl₄] · 2H₂O | 2-piperidone |

The following Examples illustrate the preparation of the indicated compounds.

### Example 1. Synthesis of compound I

Piperazine hexahydrate (2.29 g, 11.79 mmol) is dissolved in H₂O (13 ml), and the solution is acidified to pH ~1 with ECl (1.5 ml). Palladium dichloride (2.09 g, 11.78 mmol) is dissolved in the mixture of 25 ml of H₂O and 3 ml of HCl when heated (~60°C). The solutions are combined and evaporated in a water bath until crystallization begins. After cooling the resulting crystals are filtered off, washed with ethanol and dried in an oven at 90°C under constant pressure. The yield is 3.68 g, 93% of the theoretical (based on the starting palladium).
Analysis: Calculated for C₄H₁₂N₂PdCl₄, %: Pd, 31.63; Cl, 42.16. Found, %: Pd, 31.90; Cl, 41.50.

### Example 2. Synthesis of compound II

N-methyl-piperazine (1.66 g, 16.30 mmol) is dissolved in H₂O (15 ml), and the solution is acidified to pH ~1 with HCl (2.5 ml). Palladium dichloride (2.89 g, 16.30 mmol) is dissolved in the mixture of 25 ml of H₂O and 3 ml of HCl when heated, and the solution is filtered, poured into the solution of the ligand and evaporated in a water bath until crystallization begins. After cooling the resulting crystals are filtered off, washed with ethanol and dried in an oven at 90°C under constant pressure. The yield is 4.2 g, 74% of the theoretical (based on the starting palladium).
Analysis: Calculated for C₅H₁₄N₂PdCl₄, %: Pd, 30.36; Cl, 40.42. Found, %: Pd, 30.90; Cl, 41.10.

### Example 3. Synthesis of compound III

N-acetyl-piperazine (1.14 g, 1.09 mmol) is dissolved in acetone (15 ml), and the solution is acidified with HCl (2.5 ml) to protonate the ligand. Benzonitrile palladium dichloride (0.35 g, 0.91 mmol) is dissolved in acetone (15 ml) when heated, and the solution is filtered and poured into the ligand solution. The mixture is stirred using a magnetic stirrer for 3 hours. The resulting precipitate is filtered off, washed with acetone and dried in an oven at 80°C under constant pressure. The yield is 0.31 g, 74% of the theoretical (based on the starting palladium).
Analysis: Calculated for C₆H₁₄N₂OPdCl₄, %: Pd, 28.01; Cl, 37.47. Found, %: Pd, 29.12; Cl, 37.65.

### Example 4. Synthesis of compound IV

1-(2-Aminoethyl)piperazine (1.52 g, 11.77 mmol) is dissolved in of H₂O (15 ml), and the solution is acidified to pH ~1 with HCl (3.5 ml). Palladium dichloride (3.13 g, 17.65 mmol) is dissolved in the mixture of 25 ml of H₂O and 4 ml of HCl. The solutions are combined and evaporated in a water bath at 70°C until crystallization begins. After cooling the precipitated crystals are filtered off, washed with ethanol and dried in an oven at 80°C under constant pressure. The yield is 4.38 g, 74% of the theoretical (based on the starting palladium).
Analysis: Calculated for C₁₂H₃₆N₆Pd₃Cl₁₂, %: Pd, 32.16; Cl, 42.16. Found, %: Pd, 32.64; Cl, 42.80.

### Example 5. Synthesis of compound V

Morpholine (1.46 g, 16.80 mmol) is dissolved in H₂O (10 ml), and the solution is acidified to pH ~1 with HCl (2 ml). Palladium dichloride (1.49 g, 8.40 mmol) is dissolved in the mixture of 15 ml of H₂O and 2 ml of HCl when heated (55-60°C), and the solution is filtered and poured into the morpholine solution. The resulting mixture is evaporated in a boiling water bath until crystallization begins, cooled, and the resulting crystalline precipitate is filtered off from the mother liquor and dried to constant weight in an oven at 90°C under constant pressure. The yield is 3.1 g, 86.8% of the theoretical (based on the starting palladium).
Analysis: Calculated for C₈H₂₀N₂O₂PdCl₄, %: Pd, 25.06; Cl, 33.41. Found, %: Pd, 25.12; Cl, 32.52.

### Example 6. Synthesis of compound VI

Morpholine (1.17 g, 13.43 mmol) is dissolved in H₂O (15 ml), and the solution is acidified to pH ~1 with HBr (2,3 ml of 46% HBr). PdBr₂ (1.78 g, 6.71 mmol) is dissolved in the mixture of 20 ml of H₂O and 2 ml of HBr when heated (55-60°C), and the solution is filtered and poured into the ligand solution. The mixture is evaporated in a boiling water bath until crystallization begins, cooled, and the resulting crystalline precipitate is filtered off, washed with a small amount of ethanol and dried to constant weight in an oven at 90°C under constant pressure. The yield is 2.68 g, 67.5% of the theoretical (based on the starting palladium).
Analysis: Calculated for C₈H₂₀N₂O₂PdBr₄, %: Pd, 17.66; Cl, 53.06. Found, %: Pd, 17.63; Cl, 54.80.

### Example 7. Synthesis of compound VII

N-methyl-morpholine (1,84 g, 18,19 mmol) is dissolved in H₂O (20 ml), and the solution is acidified to pH ~1 with HCl (2 ml). Palladium dichloride (1,61 g, 9,08 mmol) is dissolved in the mixture of 15 ml of water and 2 ml HCl when heated (~60°C), and the solution is filtered and poured into the ligand solution. The mixture is evaporated in a boiling water bath to min volume, cooled, and the resulting crystalline precipitate is dried to constant weight in an oven at 90°C under constant pressure. The yield is 3.44 g, 84% of the theoretical (based on the starting palladium).
Analysis: Calculated for C₁₀H₂₄N₂O₂PdCl₄, %: Pd, 23.51; Cl, 31.33. Found, %: Pd, 23.30; Cl, 31.33.

### Example 8. Synthesis of compound VIII

HCl (0,21 ml) is added to 4-acetylmorpholine (0.31 g, 2.40 mmol) in acetone (5 ml). Benzonitrile palladium dichloride (0.46 g, 1.20 mmol) is dissolved in acetone (15 ml) when heated (~30°C), and the solution is filtered and poured into the ligand solution. The mixture is stirred for 5 hours, using a magnetic stirrer. The resulting precipitate is filtered off, washed with acetone and dried in an oven at 70°C under constant pressure. The yield is 0.47 g, 77% of the theoretical (based on the starting palladium).
Analysis: Calculated for C₁₂H₂₄N₂O₄PdCl₄, %: Pd, 20.91; Cl, 27.84. Found, %: Pd, 21.09; Cl, 28.51.

### Example 9. Synthesis of compound IX

4-(2-Aminoethyl)morpholine (0.95 g, 7.30 mmol) in H₂O (5 ml) is acidified to pH~1 with HCl. Palladium dichloride (1.29 g, 7.28 mmol) is dissolved when heated (~60°C) in the mixture of 15 ml of H₂O and 2 ml of HCl, and the solution is filtered and poured into the ligand solution. The mixture is evaporated in a water bath at 80°C to minimum volume, cooled, and the resulting crystalline precipitate is filtered off from the mother liquor and dried in an oven at 60°C under constant pressure. The yield is 1.85 g, 66% of the theoretical (based on the starting palladium). The title compound is isolated as monohydrate: [C₆H₁₆N₂O][PdCl₄]· H₂O.
Analysis: Calculated for C₆H₁₈N₂O₂PdCl₄, %: Pd, 27.53; Cl, 36.69. Found, %: Pd, 28.16; Cl, 36.60.

### Example 10. Synthesis of compound X

2-Piperidone (1.60 g, 16.14 mmol) is dissolved in H₂O (15 ml), and the solution is acidified to pH ~1 with HCl (2 ml). To this solution added is HCl solution of palladium dichloride (1.43 g, 8.07 mmol) in the mixture of 20 ml of H₂O and 3 ml of HCl. The mixture is evaporated in water bath until crystallization begins, cooled, and the precipitated crystals are filtered off and dried in an oven at 110°C under constant pressure. The yield is 2.32 g, 59% of the theoretical (based on the starting palladium). The title compound is crystallized with 2 molecules of water.
Analysis: Calculated for C₁₀H₂₄NO₄PdCl₄, %: Pd, 21.96; Cl, 29.96. Found, %: Pd, 21.64; Cl, 29.20.

The structures of the obtained compounds have been established by IR spectroscopy.

The infrared spectra were recorded on a Nicolet Magna-750 Fourier-transform infrared spectrometer in the 4000-100 CM⁻¹ region. The spectra in the 4000-400 cm⁻¹ region were measured with a resolution of 2 cm⁻¹ (KBr tablets). The spectra in the 600-100 cm⁻¹ region were measured with a resolution of 4 cm⁻¹ (petrolatum pastes).

The structures of the claimed compounds were determined by comparing spectra of the complexes with the spectra of free ligands (in the form of hydrochlorides), as well as of salts comprising the [PdCl₄]²⁻ anion.

The spectra of compounds V, VII, VIII, IX, X in the region of 2000-400 cm⁻¹ are similar to those of protonated ligands of the type LH⁺ in hydrochlorides L.HCl (wherein L is a heterocyclic ligand molecule). The spectra of compounds I, II and III in the same region are similar to those of corresponding cations of the type L'H₂²⁺ in dihydrochlorides, and the spectrum of compound IV is similar to that of a cation of the type L'H₃⁺³ in trihydrochloride. This unambiguously indicates that all the investigated compounds contain protonated ligands as cations. Some shifts and splits in the bonds as compared with hydrochlorides are due to the anion replacement. Significant changes in the spectra of the complexes as compared with hydrochlorides are observed in the region of 4000-2000 cm⁻¹. The most prominent change is the disappearance of a large group of strong bands with peaks in the 2450-2700 cm⁻¹ region caused by strong hydrogen bonds in solid hydrochloride. In the complexes, the system of hydrogen bonds is broken and instead of the above group of bands a strong wide band in the 3000-3100 cm⁻¹ region appears due to stretching vibrations of the type N⁺-H. For neutral molecules, there is no L absorbance in the region above 3000 cm⁻¹. The appearance of an absorption band in this region confirms that the ligand in the palladium complex is present in the protonated cationic form.

Besides the bands belonging to the protonated ligand cation and near in their position to corresponding bands in hydrochlorides, the low-frequency IR spectra (500-100 cm⁻¹) contain two new absorption bands: a strong band in the 320-335 cm⁻¹ region and a weaker band around 150-160 cm⁻¹. These bands are characteristic of the [PdCl₄]²⁻ planar square anion and assigned to the stretching vibrations of vPdCl, ν₆(E₂ᵤ), and the deformation vibrations of δPdCl₂, ν₇(Eᵤ), respectively. For comparison, in the spectrum of K₂PdCl₄ the corresponding frequencies are 321 and 161 cm⁻¹, respectively, while in the spectrum of the ionic complex with ephedrine [C₁₀H₁₆ON]₂[PdCl₄] they are observed at 323 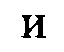 149 cm⁻¹, respectively. In going from the [PdCl₄]²⁻ anion to the [PdBr₄]²⁻ anion (in compound VI) they are shifted to 251 and 126 cm⁻¹, respectively, which confirms that they arise from palladium-haloid vibrations. It should be noted that for K₂PdBr₄ these bands are at 243 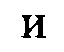 114 cm⁻¹, respectively. The absence in the low-frequency spectra of other bands that could be assigned to metal-ligand vibrations is also consistent with the ionic structure. Therefore, the IR spectra recorded in the medium-and low-frequency regions unambiguously show that the compounds of the invention have the cation-anion composition [LH⁺]₂[PdX₄²⁻] (wherein X = Cl or Br) in case of compounds V to X, [LH₂²⁺][PdCl₄²⁻] in case of compounds I to III, and [LH₃⁺³]₂[PdX₄²⁻]₃ in case of compound IV (wherein L is a corresponding ligand). This is consistent with the chemical analysis data on ligand-metal ratios in the complexes.

The positions of some of the most important absorption bands for the investigated compounds of the invention are presented in Table 2.

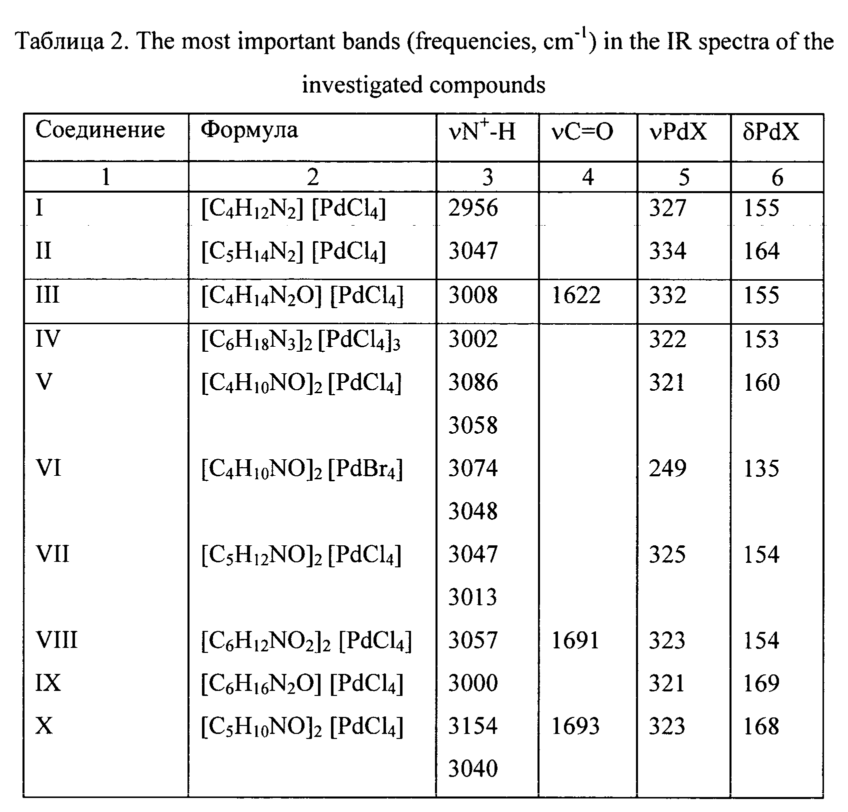

Compounds I, II, V, VI, VII, VIII, IX, X have been tested in relation to their specific activity and acute toxicity.

In the anti-tumor activity and toxicity assays, all compounds were administered to animals intravenously as solutions in 0,9% NaCl.

Leukemia P388 cells, obtained from an animal donor on day 8 of the development of leukemia, were implanted subcutaneously.

Melanoma B16 was implanted subcutaneously in the left side of a mouse. Melanoma B16 cells were obtained from animal donors on day 15.

Lewis carcinoma was implanted intramuscularly in the left hunch of a mouse. Lewis carcinoma cells were obtained from animal donors on day 15.

When implanting the tumors, suspension of their cells in saline were administered.

Female or male BDF₁, Balb or F₁ mice were used in the assays. The age of animals was within the range of 2.5-2.8 months. The weight of animals was 20-24 g.

The statistical data analysis was done using Student's test.

The results of testing the compounds on their toxicity are presented in Table 3.

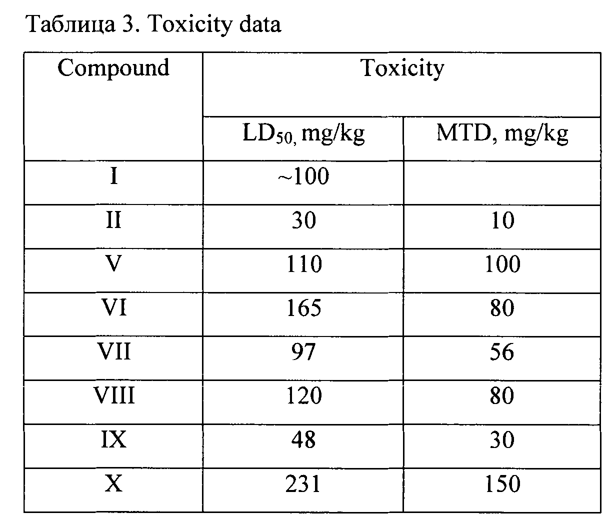

All the tested compounds are much less toxic than cis-DDP (LD₅₀ is 8 to 13 mg/kg). The LD₅₀ values for the tested compounds are within the range of 30-165 mg/kg.

The results of anti-tumor activity assays for compounds I, II, V, VI, VII, VIII and IX are presented in Table 4.

The anti-tumor activity of the compounds was tested *in vivo.* The anti-tumor activities of the tested compounds were evaluated in terms of the Tumor Growth Inhibition (TGI), Increase in Life Span (ILS) and Metastasis Inhibition Index (MII).

**Table 4.**

| Anti-tumor activity of the compounds | | | | | |
|---|---|---|---|---|---|
| Compound | Melanoma B-16 | | Lewis lung carcinoma | | Leukemia P-388 ILS, % |
| | MII, % | TGI, % | MII, % TGI, % | | |
| 1 | 2 | 3 | 4 | 5 | 6 |
| I | 61 | 38 | 83 | 41 | |
| | 20 mg/kg, days 2, 5, 8, 11, 14 | | 20 MΓ/κΓ days 2, 4, 6, 8, 10, 12 | | |
| II | 76 | 60 | | | |
| | 10 mg/kg, days 2-6 | | | | |

| 1 | 2 | 3 | 4 | 5 | 6 |
|---|---|---|---|---|---|
| V | 91 | 49 | | 37 | |
| | 28 mg/kg days 2, 5, 8, 11, 14 | | 28 mg/kg days 2, 4, 6, 8, 10, 12 | | |
| VI | 55 43 | | 94 | 56 | |
| | 28 mg/kg days 2, 5, 8, 11, 14 | | 28 mg/kg days 2, 4, 6, 8, 10, 12 | | |
| VII, early treatment, from day 2 on | 99 | 96 | | | |
| | 22 mg/kg, days 2-6 | | | | |
| VII, late treatment, from day 7 on | 98 | 22 | | | |
| | 22 mg/kg, days 7-11 | | | | |
| VIII | 99 | 9 | | | 60 |
| | 10 mg/κkg, days 2-8 | | | | |
| | 95 | 75 | | | 40 mg/kg, days 1, 4, 7, 10 |
| | 10 mg/kg, days 2, 4, 6, 8, 10, 12, 14 | | | | |
| IX | 90 | 44 | | | |
| | 8 MΓ/κΓ days 2, 4, 6, 8, 10, 12, 14 | | | | |

As can be seen from Table 4, most of the tested compounds exhibit high anti-tumor activity for melanoma B-16; compound VII exhibits high activity even in late stages of the treatment. Further, most of the tested compounds not only inhibit the growth of melanoma B-16 but also exhibit a high anti-metastatic activity. Furthermore, compound VIII increases the life span of animals having leukemia P 388 remarkably. Compounds I, V and VI also exhibit high anti-metastatic activity for Lewis lung carcinoma.

### Industrial applicability

Compounds of the invention have pharmacological activity, in particular a high anti-tumor activity; therefore they can be used in medicine.

## Claims

1. Palladium complexes with heterocyclic ligands of general formula I, 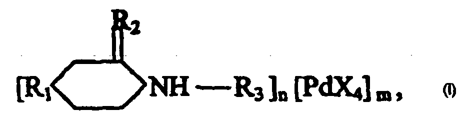 wherein
R₁ is NH₂, O or CH₂;
R₂ is 2H or O;
R₃ is H, CH₃, CH₂-CH₂-NH₃ or (CO)-CH₃;
X is Cl or Br;
when R₁ is NH₂ or O; R₂ is 2H; R₃ is H, CH₃, CH₂-CH₂-NH₃ or (CO)-CH₃, then n = 1, and m = 1;
when R₁ is O or CH₂; R₂ is O or 2H; R₃ is H, CH₃ or (CO)-CH₃, then n = 2, and m = 1;
when R₁ is NH₂; R₂ is 2H; R₃ is CH₂-CH₂-NH₃, then n = 2, and m = 3.

2. Palladium complexes with heterocyclic ligands of claim 1, **characterized in that** they have pharmacological activity.

3. Palladium complexes with heterocyclic ligands of claim 1 or claim 2, **characterized in that** they have anti-tumor activity.
